Europäisches Patentamt

European Patent Office

Office européen des brevets

(11) Publication number: **0 517 125 A1**

## EUROPEAN PATENT APPLICATION

(21) Application number: **92109169.0**

(22) Date of filing: **01.06.92**

(51) Int. Cl.5: **A61K 31/22**

(30) Priority: **05.06.91 IT RM910390**

(43) Date of publication of application:
**09.12.92 Bulletin 92/50**

(84) Designated Contracting States:
**BE CH DE DK FR GB IT LI LU NL PT SE**

(71) Applicant: **SIGMA TAU Industrie Farmaceutiche Riunite S.p.A.**
**Viale Shakespeare, 47**
**I-00144 Rome(IT)**

(72) Inventor: **Cavazzo, Claudio**
**Piazza Campitelli 2**
**Roma(IT)**

(74) Representative: **La Ciura, Salvatore**
**c/o STUDIO D'ORIO Via F. Sforza, 3**
**I-20122 Milan(IT)**

(54) **Use of propionyl L-carnitine for regaining muscle tone in immobilised patients.**

(57) The oral or parenteral administration of acyl L-carnitine, for example of propionyl L-carnitine (10-30 mg/Kg of body weight/day), allows rapid regain of muscle tone in patients suffering long periods of immobilisation, for example patients with fractures.

EP 0 517 125 A1

This invention relates to a new therapeutic use of some acyl L-carnitines to regain muscle tone in patients suffering long periods of immobilisation.

Such forms of acyl L-carnitine are acetyl, propionyl, butyryl, isobutyryl, valeryl, isovaleryl and hexanoyl L-carnitine and their pharmacologically acceptable salts. For the sake of simplicity, reference will be made below to propionyl L-carnitine. It is however intended that the teachings referring to this compound are applicable to all the acyl derivatives mentioned above.

Therapeutic uses of propionyl L-carnitine are already known in which the propionyl L-carnitine is in fact used for the treatment of cardiovascular diseases and of peripheral arteriopathies. Such uses do not in any way suggest the possibility of using propionyl L-carnitine for the purpose here described.

The use is also known of L-carnitine in the treatment of certain myopathies and muscular dystrophies. The etiological cause of these pathologies has been identified with certainty in the tissular deficiency of L-carnitine which gives rise to an excessive accumulation of lipids in the muscle fibres.

One would therefore have been able to foresee that the administration of a derivative of carnitine would have been able to compensate for the possible tissular and/or systemic deficiency caused by a prolonged period of immobilisation. However it has been found that such patients, if not suffering from primary dysmetabolic disorders, do not present with a tissular and/or systemic lack of carnitine in the period of immobilisation or later by reason of this factor.

It has therefore been unexpected and surprising to find that there is therapeutic efficacy of propionyl L-carnitine in the type of patient whose muscular pathology is not caused by tissular and/or systemic deficiency of L-carnitine, neither nor secondary with respect to the event which has caused the period of immobilisation.

For example, 3 groups of 8 patients have been studied, each of which patients had fractures of the lower limbs. Some patients were treated with L-carnitine and some with propionyl L-carnitine. Before and after a period of immobilisation of approximately the same duration (30-40 days), the circumference of the immobilised limb was measured in all 24 patients and it was noted that in the 6 treated with L-carnitine and the 6 treated with propionyl L-carnitine the difference between the two measurements was significantly less than with respect to the others (untreated). It was furthermore noted that the patients treated with propionyl L-carnitine demonstrated a still smaller reduction than those treated with L-carnitine. In such patients the reduction of tone and of muscle volume following the immobilisation was accordingly notably inhibited by the propionyl L-carnitine. Measurements of the muscular carnitine were therefore made in 3 patients per group with the object of confirming whether the immobilisation could have caused a loss of this substance.

In all patients the level was normal, so that it is possible to exclude a secondary deficiency of carnitine following upon the period of immobilisation.

The resumption of normal activity was also more rapid in the subjects treated with propionyl L-carnitine than with respect both to those treated with L-carnitine and those untreated. One may therefore deduce that propionyl L-carnitine administered to patients with traumatic fractures of the limbs inhibits the loss of muscle mass stemming from the immobilisation, favours the maintenance of muscle tone and permits a more rapid regain of the conditions preceding the trauma, accelerating the fuctional recovery of the patients.

Although the daily dose to administer depends, according to the discetion of the doctor treating the patient, upon the weight, age and general conditions of the patient, it has been found that it is generally opportune to administer from approximately 10 to approximately 30 mg of propionyl L-carnitine/Kg of body weight/day.

The propionyl L-carnitine is formulated with the usual excipients used for the preparation of compositions adapted for oral or parenteral administration.

It has further been found that a pharmaceutical composition particularly suitable for the therapeutic uses mentioned above comprises, when in the form of a single dose, from approximately 500 mg to approximately 1 000 mg of propionyl L-carnitine.

## Claims

1. The use of an acyl L-carnitine selected from acetyl, propionyl, butyryl, isobutyryl, valeryl, isovaleryl, hexanoyl L-carnitine and their pharmacologically acceptable salts, for regaining muscle tone in immobilised patients.

2. The use of an acyl L-carnitine selected from acetyl, propionyl, butyryl, isobutyryl, valeryl, isovaleryl, hexanoyl L-carnitine and their pharmacologically acceptable salts to produce a medication adapted to stimulate the regaining of muscle tone in immobilised patients.

3. The use of an acyl L-carnitine selected from acetyl, propionyl, butyryl, isobutyryl, valeryl, isovaleryl, hexanoyl L-carnitine and their pharmacologically acceptable salts comprising the oral or parenteral administration to a patient having muscle tone that is less than the norm as the result of immobilisation, of about 10.30 mg of acyl L-carnitine/Kg of body weight/day.

4. A pharmaceutical composition, in the form of a single dose, suitable for oral or parenteral administration for regaining muscle tone in immobilised patients comprising from approximately 500 mg to approximately 1 000 mg of an acyl L-carnitine selected from acetyl, propionyl, butyryl, isobutyryl, valeryl, isovaleryl, hexanoyl L-carnitine and their pharmacologically acceptable salts and a pharmacologically acceptable excipient.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | US-A-4 464 393 (C. CAVAZZA)<br>* column 2, line 43 - line 55; claims 1-2 *<br>--- | 1-4 | A61K31/22 |
| X | INT. J.CLIN. PHARM. RES.<br>vol. 10, no. 3, 1990,<br>pages 197 - 202;<br>G.G. CORBUCCI ET AL.: 'Metabolic effects induced by L-carnitine and propionyl-L-carnitine in human hypoxic muscle tissue during exercise'<br>* page 201, right column, last paragraph - page 202, left column, last paragraph *<br>--- | 1-3 | |
| X | US-A-4 320 145 (C. CAVAZZA)<br>* abstract; claims 2,3 *<br>--- | 1-3 | |
| A | US-A-4 194 006 (C. CAVAZZA)<br>* the whole document *<br>----- | 1-3 | |

TECHNICAL FIELDS
SEARCHED (Int. Cl.5 )

A61K

The present search report has been drawn up for all claims

| Place of search | Date of completion of the search | Examiner |
|---|---|---|
| MUNICH | 01 SEPTEMBER 1992 | FOERSTER W. K. |

EPO FORM 1503 03.82 (P0401)